**Europäisches Patentamt**

(19) **European Patent Office**

**Office européen des brevets**

(11) Publication number: **0 009 886**
**B1**

(12) **EUROPEAN PATENT SPECIFICATION**

(45) Date of publication of patent specification: **04.08.82**

(21) Application number: **79301797.1**

(22) Date of filing: **31.08.79**

(51) Int. Cl.³: **C 07 C 69/14,**
**C 07 C 67/08, C 07 C 69/24**

(54) **Process for the production of carboxylic acid esters employing superatmospheric pressure.**

(30) Priority: **12.09.78 GB 3641378**

(43) Date of publication of application:
**16.04.80 Bulletin 80/8**

(45) Publication of the grant of the patent:
**04.08.82 Bulletin 82/31**

(84) Designated Contracting States:
**DE FR GB IT SE**

(56) References cited:
**DE - A - 507 205**
**US - A - 1 791 238**

(73) Proprietor: **BP Chemicals Limited**
**Britannic House Moor Lane**
**London, EC2Y 9BU (GB)**

(72) Inventor: **Buttle, Denis Clive**
**BP PETROLEUM DEVELOPMENT LIMITED**
**Britannic House**
**Moor Lane London, EC2Y 9BU (GB)**

(74) Representative: **Harry, John et al,**
**BP INTERNATIONAL LIMITED Patents and**
**Licensing Division Chertsey Road**
**Sunbury-on-Thames Middlesex TW16 7LN (GB)**

Process for the production of carboxylic acid esters employing superatmospheric pressure

The present invention relates to a process for the production of carboxylic acid esters and in particular to a process for the production of ethyl acetate.

DE 507205 describes a process for the production of methyl and ethyl acetate characterised by the fact that one heats the mixture of acids and alcohols, possibly in dilute solutions, dispensing with the participation of catalysts, to temperatures above 100°C, for example temperatures of 150 to 200°, with the application of pressure, and the resultant esters are continually distilled off from the reaction mixture.

USP 1 791 238 describes a process for the liquid phase production of esters of the lower members of the fatty acid series, consisting in causing mixtures of fatty acids and alcohols to react upon one another at temperatures of about 100 to 180°C, corresponding to working pressures of 2 to 10 atmospheres in the presence of catalysts which are capable of producing moderate hydrogen-ion concentrations and removing the ester formed continuously from the reaction mixture.

A conventional process for the production of volatile esters of a primary alcohol and a carboxylic acid consists of continuously feeding the alcohol and the acid in substantially equimolar quantities to a reaction still operated at atmospheric pressure. The reaction still invariably contains an esterification catalyst in the form of a strong mineral acid such as sulphuric acid. The ester produced thereby is recovered by distilling overhead an ester/water azeotrope. An essentially anhydrous composition is maintained in the reaction still by returning ester product phase, separated by decantation from the aqueous product, to the top of the column to give a reflux ratio of about 3:1. Such a process is described in the complete specification of our British Patent No. 1173089. Virtually complete conversion of alcohol to ester is thereby achieved and the ester product typically contains ⩽2% w/w alcohol which simplifies its downstream purification. On the other hand the use of such a high rate of reflux return is undesirable because the production capacity of the reaction still is approximately inversely proportional to the reflux ratio used. It is therefore a desirable objective to reduce the reflux ratio whilst maintaining high conversion and low alcohol content in the ester product and, as a result, correspondingly increase the production capacity of the still.

Vapour/liquid equilibrium data reported by Merriman et al in JCS, p 1797 and p 1814, (1913) indicates that the water content of the ethyl acetate:ethanol:water azeotropes is increased from ca 9 to 10% w/w by increasing the pressure from 1 bar to 2 bars. It would therefore be expected that increasing the pressure would correspondingly increase the water content of the overhead ester product which would permit a corresponding reduction in the minimum reflux ratio required to remove water overhead whilst maintaining the reaction mixture essentially anhydrous and would correspondingly increase the production capacity of the still. The effect of increasing pressure on the kinetics of the reaction is not reported but the same vapour/liquid equilibrium data indicate that the ethanol content of the ternary azeotrope is also increased from 8.4 to 12.1% w/w by increasing the pressure from 1 to 1.93 bars. It may therefore also be expected that increasing the pressure would correspondingly and disadvantageously increase the alcohol content of the overhead ester product thus increasing the difficulty in downstream purification of the

We have now found that superatmospheric pressure operation of the esterification reaction still surprisingly leads to no substantial increase in the alcohol content of the ester product removed overhead. Furthermore the vapour velocity in the column is correspondingly decreased, thereby potentially allowing a further significant increase in ester production rate. Moreover the increase in production rate at the higher still pressures observed in the production of ethyl acetate can be extended to the production of other esters which form heterogeneous azeotropes with water and which have a lower boiling point than water.

Accordingly the present invention provides a process for the production of a carboxylic acid ester by continuously feeding either ethanol, n-propanol or iso-propanol and a substantially equimolar quantity of either acetic, propionic or butyric acid to an esterification reaction still containing an esterification catalyst which still is maintained under conditions resulting in the formation of ester product, removing the ester product and water formed in the reaction overhead by distillation and returning a proportion of the ester product as reflux to the distillation section of the esterification reaction still so as to maintain an essentially anhydrous reaction composition in the kettle of the reaction still, characterised in that the esterification still is maintained under superatmospheric pressure.

Although the alcohol may be ethanol, n-propanol or iso-propanol the preferred alcohol is ethanol. Whilst the carboxylic acid may be acetic acid, propionic acid or butyric acid the preferred carboxylic acid is acetic acid.

The esterification catalyst is preferably a strong mineral acid such as sulphuric acid or toluene-para-sulphonic acid. The concentration of the mineral acid catalyst in the esterification reaction still may suitably be >0.5 w/w, preferably in the range from 1 to 5% w/w of the esterification reaction mixture.

An esterification reaction still typically comprises a reaction kettle surmounted by a distillation column provided with conventional overhead take-off and reflux facilities. The ester product phase is preferably separated from the water phase by decantation. The esterification reaction temperature may suitably be $>80°C$. Generally, and particularly in the case of ethyl acetate production, the temperature is preferably in the range from 110 to 160°C.

The superatmospheric pressure applied to the esterification reaction still may suitably be in the range 1.38 to 13.8 bars, preferably 1.72 to 3.10 bars. The superatmospheric pressure may suitably be provided by introduction of an inert gas, e.g. nitrogen, from a high pressure source or by the production of autogenous pressure, suitably by insertion of a pressure control valve in the vapour pipe between the head of the column and the condenser.

Using superatmospheric pressure, reflux ratios of less than 3:1 are preferably employed.

The invention will now be illustrated by reference to the to the following Examples.

Comparison Test

An esterification feed mixture of acetic acid (117 moles) and aqueous ethanol (120 moles of ethanol containing 6.5% w/w water) was fed during 24 hours into a reboiler (whose contents [1.2 kg] comprised 83.9% w/w acetic acid, 9.7% w/w ethyl acetate, 3.6% w/w toluene-para-sulphonic acid and 2.8% w/w of water) which was connected to a 25 plate Oldershaw column (50 mm diameter) fitted with an overhead condenser/decanter. This esterification was carried out at atmospheric pressure. The overhead product was cooled and the two phases formed were separated in the decanter. The ester phase was recycled into the top of the column to give a reflux ratio of 3.1:1. Under these conditions the boil-up rate within the column amounted to 90% of its flood point. The ester product composition (10.6 kg of oil phase comprising 95.9% w/w ethyl acetate, 0.9% w/w ethanol and 3.2% w/w water and 2.35 kg of aqueous phase comprising 7.8% w/w ethyl acetate, 2.6% w/w ethanol and 89.5% w/w water) corresponds to 97.2% conversion of ethanol to ester. This is not an example of the process of the present invention because the pressure applied to the esterification reaction still was not superatmospheric.

Example 1

The previous procedure was repeated except that the reaction still was operated at 1.72 bars which was achieved by pressurising with nitrogen gas. An esterification reaction feed mixture of acetic acid (79.2 moles) and aqueous ethanol (81.3 moles containing 6.5% w/w water) was fed into the reaction mixture during 13 hours and ester product was collected overhead using a reflux ratio of 2.92:1. Under these conditions the boil-up rate within the column amounted to 90% of its flood point. The amount and composition of the ester product (7.14 kg of oil phase comprising 95% w/w ethyl acetate, 1.5% w/w ethanol and 3.5% w/w water and 1.62 kg of water phase comprising 8% w/w ethyl acetate, 4.1% w/w ethanol and 87.9% w/w water) corresponded to 95.4% conversion of ethanol to ester. This rate of production of ester was 23% greater than the maximum achievable in the Comparison Test by operation at atmospheric pressure.

Example 2

The procedure used in the Comparison Test was repeated, except that the reaction still was operated at 2.07 bars which was achieved by pressurising with nitrogen. The reaction conditions and results are given in the following Table.

Example 3

Example 2 was repeated under the conditions and with the results shown in the following Table.

Approximately half the expansion in ester production obtained in Example 3 was theoretically attributably to the increase in water content of the overhead ester product and the remainder resulted from the decrease in vapour velocity obtained by the use of superatmospheric pressure.

Although the ethanol content of the ester product obtained in Example 3 increased slightly from 0.9% w/w (the base line ester product) to 1.6% w/w this did not significantly affect the production of water-/alcohol-free ethyl acetate in the downstream purification stills.

TABLE

| Example No. | Esterification Pressure (bars) | Esterification Kettle Temperature (°C) | Water Content (% w/w) | AcOH/EtOH Feed Rate $(cm^3 \cdot h^{-1})$ | Reflux Ratio Used | % Increase in Ester Production (a) | % Conversion of Ethanol to Ethyl Acetate |
|---|---|---|---|---|---|---|---|
| Comparison Test | 1.03 | 109 | 4 | 576 | 3.1 | Base-line | 97.2 |
| 2 | 2.07 | 130 | 4.4 | 915 | 2.5 | 57 | 95.9 |
| 3 | 2.07 | 129 | 7.1 | 1,040 | 1.87 | 77 | 95.5 |

NOTES

(a)  As determined at ca. 90% of esterification column flood point.

## Claims

1. A process for the production of a carboxylic acid ester by continuously feeding either ethanol, *n*-propanol or *iso*-propanol and a substantially equimolar quantity of either acetic, propionic or butyric acid to an esterification reaction still containing an esterification catalyst which still is maintained under esterification reaction conditions resulting in the formation of ester product, removing the ester product and water formed in the reaction overhead by distillation and returning a proportion of the ester product as reflux to the distillation section of the esterification reaction still so as to maintain an essentially anhydrous reaction composition in the kettle of the reaction still, characterised in that the esterification reaction still is maintained under superatmospheric pressure.

2. A process according to claim 1 wherein ethanol is fed to the esterification reaction still.

3. A process according to either one of the preceding claims wherein acetic acid is fed to the esterification reaction still.

4. A process according to any one of the preceding claims wherein the esterification catalyst is a strong mineral acid.

5. A process according to claim 4 wherein the mineral acid is sulphuric acid or toluene-para-sulphonic acid.

6. A process according to either one of claims 4 and 5 wherein the concentration of the mineral acid catalyst in the esterification reaction still is in the range from 1 to 5% w/w of the esterification reaction mixture.

7. A process according to any one of the preceding claims wherein the esterification reaction temperature is in the range from 110 to 160°C.

8. A process according to any one of the preceding claims wherein the superatmospheric pressure is in the range from 1.38 to 13.8 bars.

9. A process according to claim 8 wherein the superatmospheric pressure is in the range from 1.72 to 3.10 bars.

10. A process according to any one of the preceding claims wherein the reflux ratio is less than 3:1.

11. A process according to any one of the preceding claims wherein the superatmospheric pressure is provided by introduction of an inert gas from a high pressure source.

12. A process according to any one of claims 1 to 10 wherein the superatmospheric pressure is produced autogenously.

## Revendications

1. Procédé pour produire un ester d'acide carboxylique par introduction continue de l'éthanol, de *n*-propanol ou de l'isopropanol et d'une quantité sensiblement équimolaire de l'acide acétique, propionique, ou butyrique dans un appareillage de réaction d'estérification contenant un catalyseur d'estérification, appareillage qui est maintenu dans les conditions de réaction d'estérification provoquant la formation d'un ester comme produit, enlèvement par distillation, en tête, de l'ester produit et de l'eau formée dans la réaction, et retour d'une certaine proportion de l'ester produit, comme reflux renvoyé à la section de distillation de l'appareillage de la réaction d'estérification, afin de maintenir une composition de réaction essentiellement anhydre dans la chaudière de l'appareillage de réaction, procédé caractérisé en ce que l'appareillage de la réaction d'estérification est maintenu sous une pression supérieure à la pression atmosphérique.

2. Procédé selon la revendication 1, dans lequel de l'éthanol est introduit dans l'appareillage de la réaction d'estérification.

3. Procédé selon l'une ou l'autre des revendications précédentes, dans lequel de l'acide acétique est introduit dans l'appareillage de la réaction d'estérification.

4. Procédé selon l'une quelconque des revendications précédentes, dans lequel le catalyseur d'estérification est un acide minéral fort.

5. Procédé selon la revendication 4, dans lequel l'acide minéral est l'acide sulfurique, ou l'acide para-toluène-sulfonique.

6. Procédé selon l'une des revendications 4 et 5, dans lequel la concentration de l'acide minéral servant de catalyseur dans l'appareillage de la réaction d'estérification se situe dans la gamme de 1 à 5% en poids/poids du mélange de la réaction d'estérification.

7. Procédé selon l'une quelconque des revendications précédentes, dans lequel la température de la réaction d'estéfication se situe dans la gamme de 110°C à 160°C.

8. Procédé selon l'une quelconque des revendications précédentes, dans lequel la pression supérieure à la pression atmosphérique se situe dans la gamme de 1,38 à 13,8 bars.

9. Procédé selon la revendication 8, dans lequel la pression supérieure à la pression atmosphérique se situe dans la gamme de 1,72 à 3,10 bars.

10. Procédé selon l'une quelconque des revendications précédentes, dans lequel le taux de reflux est inférieur à 3:1.

11. Procédé selon l'une quelconque des revendications précédentes, dans lequel la pression supérieure à la pression atmosphérique est obtenue par introduction d'un gaz inerte provenant d'une source sous pression élevée.

12. Procédé selon l'une quelconque des revendications 1 à 10, dans lequel la pression supérieure à la pression atmosphérique est produite de manière autogène.

## Patentansprüche

1. Verfahren zur Herstellung von Carbonsäureestern durch kontinuierliche Einführung von entweder Äthanol, n-Propanol oder Iso-Propanol und von einer im wesentlichen äquimolaren Menge von entweder Essigsäure, Propionsäure oder Buttersäure in ein Veresterungsgefäß, welches einen Veresterungskatalysator enthält, wobei das Veresterungsgefäß unter Veresterungsbedingungen gehalten wird, die zur Bildung des Esterproduktes führen, das Esterprodukt und das bei der Reaktion gebildete Wasser durch Destillation überkopf abgeführt werden und ein Teil des Esterproduktes als Rücklauf in den Destillationsteil des Veresterungsgefäßes zurückgeführt wird, so daß ein im wesentlichen wasserfreies Reaktionsmilieu in dem Kessel des Reaktionsgefäßes aufrechterhalten wird, dadurch gekennzeichnet, daß das Veresterungsgefäß unter überatmosphärischem Druck gehalten wird.

2. Verfahren gemäß Anspruch 1, wobei Äthanol in das Veresterungsgefäß eingeführt wird.

3. Verfahren gemäß einem der vorgehenden Ansprüche, wobei Essigsäure in das Veresterungsgefäß eingeführt wird.

4. Verfahren gemäß einem der voreghenden Ansprüche, wobei der Veresterungskatalysator eine starke Mineralsäure ist.

5. Verfahren gemäß Anspruch 4, wobei die Mineralsäure Schwefelsäure oder p-Toluolsulfonsäure ist.

6. Verfahren gemäß einem der Ansprüche 4 und 5, wobei die Konzentration des Mineralsäurekatalysators in dem Veresterungsgefäß im Bereich von 1 bis 5 Gew.-% bezogen auf die Veresterungs-Reaktionsmischung beträgt.

7. Verfahren gemäß einem der vorgehenden Ansprüche, wobei die Veresterungstemperatur im Bereich von 110 bis 160°C liegt.

8. Verfahren gemäß einem der vorgehenden Ansprüche, wobei der überatmosphärische Druck im Bereich von 1,38 bis 13,8 Bar liegt.

9. Verfahren gemäß Anspruch 8, wobei der überatmosphärische Druck im Bereich von 1,72 bis 3,10 Bar liegt.

10. Verfahren gemäß einem der vorgehenden Ansprüche, wobei das Rückflußverhältnis geringer als 3:1 ist.

11. Verfahren gemäß einem der vorgehenden Ansprüche, wobei der überatmosphärische Druck durch Einführung eines Inertgases aus einer Hochdruckquelle hergestellt wird.

12. Verfahren gemäß einem der Ansprüche 1 bis 10, wobei der überatmosphärische Druck autogen erzeugt wird.